# EUROPÄISCHE PATENTSCHRIFT

(11) **EP 0 633 889 B1**
(45) Veröffentlichungstag und Bekanntmachung des Hinweises auf die Patenterteilung: **05.03.1997**
(21) Anmeldenummer: 93907833.3
(22) Anmeldetag: 25.03.1993
(51) Int. Cl.: C07H 15/04

(54) **ALKYL- UND/ODER ALKENYLOLIGOGLYKOSIDCARBONATE**
ALKYL-OLIGOGLYCOSIDE AND/OR ALKENYL-OLIGOCLYCOSIDE CARBONATES
CARBONATES D'OLIGOGLUCOSIDES D'ALKYLE ET/OU D'ALCENYLE

(30) Priorität: 02.04.1992 DE 4210913
(43) Veröffentlichungstag der Anmeldung: 18.01.1995
(73) Patentinhaber: Henkel Kommanditgesellschaft auf Aktien, 40191 Düsseldorf (DE)
(72) Erfinder: WEUTHEN, Manfred, D-5650 Solingen 11 (DE)
(86) Internationale Anmeldenummer: EP9300725
(87) Internationale Veröffentlichungsnummer: WO9320089

(56) Entgegenhaltungen:
- DATABASE WPI Derwent Publications Ltd., London, GB; AN 78-29293A

## Beschreibung

### Gebiet der Erfindung

Gegenstand der Erfindung sind Alkyl- und/oder Alkenyloligoglykosidcarbonate, ein Verfahren zu ihrer Herstellung sowie ihre Verwendung zur Herstellung oberflächenaktiver Mittel.

### Stand der Technik

Seit einer Reihen von Jahren hat sich der Trend verstärkt, Spezialchemikalien für Wasch- und Reinigungsmittel auf der Grundlage von nachwachsenden Rohstoffen wie etwa Zucker oder Stärke herzustellen. Ein Beispiel hierfür sind die Alkyloligoglykoside, die beispielsweise eine Alternative zu herkömmlichen nichtionischen Tensiden auf Basis von Fettalkohol und Ethylenoxid darstellen können. Für manche kosmetischen Anwendungen sind die Eigenschaften der Alkyloligoglykoside jedoch nicht völlig befriedigend. An ihrer Stelle werden daher vorzugsweise Ester der Alkyloligoglykoside mit Carbonsäuren unterschiedlicher Kettenlänge eingesetzt **[Seifen-Öle-Fette-Wachse, 117, 124 (1991)]**.

Die Herstellung solcher Alkyloligoglykosidester ist jedoch mit zwei Problemen behaftet:
1. die thermische Instabilität des Glykosidkörpers unter den Veresterungsbedingungen mit der Gefahr der Karamelisierung sowie
2. die unzureichend Löslichkeit der Alkyloligoglykoside in gängigen Lösungsmitteln.

In der Vergangenheit hat es nicht an Versuchen gefehlt, technische Lösungen für die geschilderten Probleme zur Verfügung zu stellen:

So berichten beispielsweise Gibbons und Swanson in **J.Am.Oil**. **Chem.Soc., 36, 553 (1959)** über ihre Versuche zur Herstellung von Mono- und Diestern aus Methylglucosid und Fettsäuren in Gegenwart von Natriumhydroxid, Bleioxid und Zinnseife im Lösungsmittel Xylol. Ein Verfahren unter Beteiligung von Xylol und Bleiverbindungen ist für die Herstellung kosmetischer Produkte aus toxikologischer Sicht jedoch äußerst bedenklich.

Unbefriedigend verliefen die Versuche von Albano-Garcia und Lorica, die Veresterung in Gegenwart von sauren und basischen Katalystoren in DMF zu wiederholen: die Reaktionsprodukte waren inhomogen und karamelisierten. Auch die Umesterung von Kokosöl mit Methylglucosiden unter Verwendung von metallischem Natrium und Kaliumseife als Katalysatoren lieferte dunkel gefärbte Produkte, die eine Reinigung mit Ethylacetat und Wasser erforderlich machte **[J.Coconut.Stud.**, **5(1), 51** **(1980)**]. Für eine technische Realisierung kommt ein derartig aufwendiges Verfahren freilich ebenfalls nicht in Betracht.

Über die direkte Veresterung von Fettsäurechloriden mit Methylgluosid berichtet schließlich Zedlinski in **Bull.Acad. Polon, Sci. 9, 103 (1961)**. Die Verwendung von Fettsäurechloriden stellt jedoch hohe Anforderungen an den Arbeitsschutz und ist aus diesem Grunde unerwünscht.

Die Aufgabe der Erfindung bestand somit darin, neue Alkyl- und/oder Alkenyloligoglucosidester zur Verfügung zu stellen, deren Herstellung nicht mit den geschilderten Nachteilen behaftet ist.

### Beschreibung der Erfindung

Gegenstand der Erfindung sind Alkyl- und/oder Alkenyloligoglykosidcarbonate, dadurch erhältlich, daß man Alkyl- und/ oder Alkenyloligoglykoside der Formel (I),

R¹O-(R²O)ₓ-[G]ₚ (I)

in der
- R¹: für einen Alkyl- und/oder Alkenylrest mit 1 bis 22 Kohlenstoffatomen,
- R²: für einen Alkylenrest mit 2 bis 4 Kohlenstoffatomen,
- x: für 0 oder Zahlen von 1 bis 30,
- [G]: für einen Zuckerrest mit 5 oder 6 Kohlenstoffatomen und
- p: für Zahlen von 1 bis 10
steht, in Gegenwart basischer Katalysatoren mit Dialkylcarbonaten der Formel (II)

R³O-CO-OR⁴ (II)

in der
- R³ und R⁴: unabhängig voneinander für Alkylreste mit 1 bis 10 Kohlenstoffatomen
stehen, kondensiert.

Die neuen Alkyl- und/oder Alkenyloligoglykosidester stellen somit Kohlensäureester der Alkyl- und/oder Alkenyloligoglykoside dar. Überraschenderweise wurde gefunden, daß die Kondensation von Alkyl- und/oder Alkenyloligoglykosiden mit Dialkylcarbonaten unabhängig von der Kettenlänge der Ausgangsstoffe praktisch vollständig und ohne Verfärbung der Reaktionsprodukte verläuft. Das Problem der mangelhaften Löslichkeit kann zudem überwunden werden, indem man die Dialkylcarbonate im Überschuß und somit gleichzeitig als Umesterungskomponente und als Lösungsmittel einsetzt.

Ein weiterer Gegenstand der Erfindung betrifft ein Verfahren zur Herstellung von Alkyl- und/oder Alkenyloligoglykosidcarbonaten, bei der man Alkyl- und/oder Alkenyloligoglykoside der Formel (I),

R¹O-(R²O)ₓ-[G]ₚ (I)

in der
- R¹: für einen Alkyl- und/oder Alkenylrest mit 1 bis 22 Kohlenstoffatomen,
- R²: für einen Alkylenrest mit 2 bis 4 Kohlenstoffatomen,
- x: für 0 oder Zahlen von 1 bis 30,
- [G]: für einen Zuckerrest mit 5 oder 6 Kohlenstoffatomen und
- p: für Zahlen von 1 bis 10
steht, in Gegenwart basischer Katalysatoren mit Dialkylcarbonaten der Formel (II)

R³O-CO-OR⁴ (II)

in der
- R³ und R⁴: unabhängig voneinander für Alkylreste mit 1 bis 10 Kohlenstoffatomen
stehen, kondensiert.

**Alkyl- und/oder Alkenyloligoglykoside,** die als Einsatzstoffe für die Herstellung der erfindungsgemäßen Alkyl- und/oder Alkenyloligoglykosidcarbonate in Betracht kommen, stellen bekannte Stoffe dar, die nach den einschlägigen Verfahren der präparativen organischen Chemie zugänglich sind. Ein Verfahren zu ihrer Herstellung stellt beispielsweise die sauer katalysierte Acetalisierung von Glucose mit Fettalkoholen dar. Stellvertretend für das umfangreiche Schrifttum sei auf die Europäische Patentanmeldung **EP-A1-0 301 298** verwiesen.

Bevorzugt sind Alkyl- und/oder Alkenyloligoglykoside, die sich von Aldosen bzw. Ketosen und wegen ihrer leichten Verügbarkeit insbesondere von der Glucose ableiten. Die bevorzugten Alkyloligoglykoside sind somit die Alkyloligoglucoside.

Die Indexzahl p in der allgemeinen Formel (I) gibt den Oligomerisierungsgrad (DP-Grad), d. h. die Verteilung von Mono- und Oligoglykosiden an und steht für eine Zahl zwischen 1 und 10. Während p in einer gegebenen Verbindunge stets ganzzahlig sein muß und hier vor allem die Werte p = 1 bis 6 annehmen kann, ist der Wert p für ein bestimmtes Alkyl- und/oder Alkenyloligoglykosid eine analytisch ermittelte rechnerische Größe, die meistens eine gebrochene Zahl darstellt. Bevorzugt sind Alkyloligoglykoside mit einem mittleren Oligomerisierungsgrad p von 1,1 bis 3,0; besonders bevorzugt sind solche Alkyl- und/oder Alkenyloligoglykoside, deren Oligomerisierungsgrad kleiner als 1,7 ist und insbesondere zwischen 1,2 und 1,4 liegt.

Der Rest R¹ kann sich von gesättigten und/oder ungesättigten primärem Alkoholen mit 8 bis 22, vorzugsweise 8 bis 10 bzw. 12 bis 18 Kohlenstoffatomen ableiten. Typische Beispiele sind Methanol, Butanol, Capronalkohol, Caprinalkohol, 2-Ethylhexanol, Caprylalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Arachylalkohol, Gadoleylalkohol, Behenylalkohol und Erucylalkohol sowie technische Schnitte, die diese Alkohole in unterschiedlichen Mengen enthalten können.

Die Alkyl- und/oder Alkenyloligoglykoside können ferner in Form ihrer Addukte mit 1 bis 30 Mol Ethylen-, Propylen- und/oder Butylenoxid vorliegen.

Alkyl- und/oder Alkenyloligoglykosidcarbonate mit besonders vorteilhaften anwendungstechnischen Eigenschaften werden erhalten, wenn man Alkyloligoglykoside der Formel (I) einsetzt, in der R¹ für einen Alkylrest mit 8 bis 18 Kohlenstoffatomen, x für 0, [G] für einen Glucoserest und p für Zahlen von 1,3 bis 3 steht. Besonders bevorzugt sind C₈-C₁₀- sowie C₁₂-C₁₈-Alkyloligoglucoside.

Auch bei den **Dialkylcarbonaten** handelt es sich um bekannte Verbindungen, die nach einschlägigen Verfahren der organischen Chemie erhalten werden können. Technisch relevant und daher im Sinne der Erfindung bevorzugt, ist der Einsatz von Dialkylcarbonaten der Formel (II), in der R³ und R⁴ für Methyl- und/oder Ethylreste stehen. Zu Herstellung und physikalischen Eigenschaften dieser Produkte sei beispielsweise auf **Chim.L'Industr., 18 (1990)** verwiesen.

Die Auswahl der **basischen Katalysatoren** ist unkritisch. Vorzugsweise werden jedoch Alkalien eingesetzt, die ausgewählt sind aus der Gruppe, die von Alkali- und Erdalkalimetalloxide, -hydroxiden, -carbonaten und -C₁-C₄-alkylaten gebildet wird. Typische Beispiele sind Natriumhydroxid, Natriumcarbonat Kaliumhydroxid, Calciumoxid, Natriummethylat und Kalium-tert.butylat. Die basischen Katalysatoren können in Mengen von 0,1 bis 5, vorzugsweise 1 bis 3 mol-% - bezogen auf die Alkyl- und/oder Alkenyloligoglykoside - eingesetzt werden.

Die Alkyl- und/oder Alkenyloligoglykosiden und die Dialkylcarbonate können in einem molaren Verhältnis von 1 : 1 bis 1 : 15 eingesetzt werden. Für eine gute Durchmischung der Reaktanten hat es sich als vorteilhaft erwiesen, die Dialkylcarbonate im Überschuß einzusetzen und somit gleichzeitig als Umesterungskomponente und als Lösungsmittel zu verwenden. Besonders bevorzugt ist daher ein molares Verhältnis von 1 : 4 bis 1 : 12.

Für eine rasche und möglichst vollständige Kondensation hat es sich als ausreichend erwiesen, die Reaktion bei der Siedetemperatur des Dialkylcarbonats durchzuführen. In einer bevorzugten Ausführungsform der Erfindung erfolgt die Umesterung mit Diethylcarbonat bei einer Temperatur im Bereich von 110 bis 120°C.

Die Umsetzung der Alkyl- und/oder Alkenyloligoglykosiden mit den Dialkylcarbonaten stellt eine Umesterung dar, bei der eine oder mehrere Hydroxylgruppen des Glucosids mit einer oder auch beiden Estergruppen des Dialkylcarbonats unter Freisetzung von Alkohol abreagieren. Der Natur der Kondensationsreaktion folgend, wird ein komplexes Gemisch erhalten, das jedoch überwiegend Glucosidmonoester und in geringeren Mengen Diester enthält. Nach Abschluß der Reaktion kann das basische Rohprodukt neutralisiert und überschüssiges Dialkylcarbonat sowie im Verlauf der Kondensation gebildeter Alkohol im Vakuum abdestilliert werden.

In einer besonderen Ausführungsform des erfindungsgemäßen Verfahrens wird bereits während der Kondensation ein Teil des gebildeten Alkohols kontinuierlich aus dem Gleichgewicht entfernt, beispielweise durch Destillation. Auf diese Weise wird eine Verschiebung des Gleichgewichtes bewirkt und Produkte erhalten, die sich durch einen hohen Anteil an Diestern auszeichnen.

### Gewerbliche Anwendbarkeit

Die erfindungsgemäßen Alkyl- und/oder Alkenyloligoglykosidcarbonate besitzen oberflächenaktive Eigenschaften. Sie erweisen sich als dermatologisch gut verträglich und leicht konfektionierbar.

Ein weiterer Gegenstand der Erfindung betrifft daher ihre Verwendung zur Herstellung von Wasch-, Spül- und Reinigungsmitteln sowie Produkten der Haar- und Körperpflege, in denen sie in Mengen von 0,1 bis 50, vorzugsweise 1 bis 25 Gew.-%-bezogen auf die Mittel - enthalten sein können.

Die folgenden Beispiele sollen den Gegenstand der Erfindung näher erläutern, ohne ihn darauf einzuschränken.

### Beispiele

### Beispiel 1:

**Alkyloligoglucosid-monoethylcarbonat**. In einem 2-l-Dreihalskolben mit Rührer, Rückflußkühler und Destillationsaufsatz wurden 384 g (1 mol) Dodecyloligoglucosid (DP = 1,2), 1180 g (10 mol) Diethylcarbonat und 0,02 mol Natriumhydroxid vorgelegt und unter intensivem Rühren über einen Zeitraum von 1 h unter Rückfluß (ca. 120°C) erhitzt. Anschließend wurde die Reaktionsmischung mit Phosphorsäure neutralisiert und überschüssiges Diethylcarbonat sowie aus der Kondensation stammendes Ethanol im Vakuum abdestilliert. Die Ausbeute betrug 414 g entsprechend 91 % der theoretischen Menge. Das Alkyloligoglucosid-monoethylcarbonat wurde anschließend bei 80°C mit 95 g Wasser zu einer Paste verarbeitet.

### Beispiel 2:

**Alkyloligoglucosid-di(monethylcarbonat).** Beispiel 1 wurde wiederholt, jedoch bereits während der Reaktion 61 g (0,8 mol) Ethanol abdestilliert. Anschließend wurde das Produkt neutralisiert und Diethylcarbonat sowie das restliche gebildete Ethanol abdestilliert. Die Ausbeute betrug 475 g entsprechend 91 % der theoretischen Menge.

## Patentansprüche

1. Alkyl- und/oder Alkenyloligoglykosidcarbonate, dadurch erhältlich, daß man Alkyl- und/oder Alkenyloligoglykoside der Formel (I),
R¹O-(R²O)ₓ-[G]ₚ (I)
in der
R¹ für einen Alkyl- und/oder Alkenylrest mit 1 bis 22 Kohlenstoffatomen,
R² für einen Alkylenrest mit 2 bis 4 Kohlenstoffatomen,
x für 0 oder Zahlen von 1 bis 30,
[G] für einen Zuckerrest mit 5 oder 6 Kohlenstoffatomen und
p für Zahlen von 1 bis 10
steht, in Gegenwart basischer Katalysatoren mit Dialkylcarbonaten der Formel (II)
R³O-CO-OR⁴ (II)
in der
R³ und R⁴ unabhängig voneinander für Alkylreste mit 1 bis 10 Kohlenstoffatomen
stehen, kondensiert.

2. Verfahren zur Herstellung von Alkyl- und/oder Alkenyloligoglykosidcarbonaten, bei man Alkyl- und/oder Alkenyloligoglykoside der Formel (I),
R¹O-(R²O)ₓ-[G]ₚ (I)
in der
R¹ für einen Alkyl- und/oder Alkenylrest mit 1 bis 22 Kohlenstoffatomen,
R² für einen Alkylenrest mit 2 bis 4 Kohlenstoffatomen,
x für 0 oder Zahlen von 1 bis 30,
[G] für einen Zuckerrest mit 5 oder 6 Kohlenstoffatomen und
p für Zahlen von 1 bis 10
steht, in Gegenwart basischer Katalysatoren mit Dialkylcarbonaten der Formel (II)
R³O-CO-OR⁴ (II)
in der
R³ und R⁴ unabhängig voneinander für Alkylreste mit 1 bis 10 Kohlenstoffatomen
stehen, kondensiert.

3. Verfahren nach Anspruch 2, **dadurch gekennzeichnet,** daß man Alkyloligoglykoside der Formel (I) einsetzt, in der R¹ für einen Alkylrest mit 8 bis 18 Kohlenstoffatomen, x für 0, [G] für einen Glucoserest und p für Zahlen von 1,3 bis 3 steht.

4. Verfahren nach Anspruch 2, **dadurch gekennzeichnet,** daß man Dialkylcarbonate der Formel (II) einsetzt, in der R³ und R⁴ für Methyl- und/oder Ethylreste stehen.

5. Verfahren nach Anspruch 2, **dadurch gekennzeichnet,** daß man basische Katalysatoren einsetzt, die ausgewählt sind aus der Gruppe, die von Alkali- und Erdalkalimetalloxiden, -hydroxiden, -carbonaten und -C₁-C₄-alkylaten gebildet wird.

6. Verfahren nach Anspruch 2, **dadurch gekennzeichnet,** daß man die basischen Katalysatoren in Mengen von 0,1 bis 5 mol-% - bezogen auf die Alkyl- und/oder Alkenyloligoglykoside - einsetzt.

7. Verfahren nach Anspruch 2, **dadurch gekennzeichnet,** daß man die Alkyl- und/oder Alkenyloligoglykosiden und die Dialkylcarbonate in einem molaren Verhältnis von 1 : 1 bis 1 : 15 einsetzt.

8. Verfahren nach Anspruch 2, **dadurch gekennzeichnet,** daß man die Kondesation bei der Siedetemperatur des Dialkylcarbonats durchführt.

9. Verfahren nach Anspruch 2, **dadurch gekennzeichnet,** daß man im Verlauf der Kondensation gebildeten Alkohol kontinuierlich aus dem Gleichgewicht entfernt.

10. Verwendung von Alkyl- und/oder Alkenyloligoglykosidcarbonaten nach Anspruch 1 zur Herstellung oberflächenaktiver Mittel.

## Claims

1. Alkyl and/or alkenyl oligoglycoside carbonates obtainable by condensing alkyl and/or alkenyl oligoglycosides corresponding to formula (I):
R¹O-(R²O)ₓ-[G]ₚ (I)
in which
R¹ is an alkyl and/or alkenyl group containing 1 to 22 carbon atoms,
R² is an alkylene group containing 2 to 4 carbon atoms,
x is 0 or a number of 1 to 30,
[G] is a sugar unit containing 5 or 6 carbon atoms and
p is a number of 1 to 10,
with dialkyl carbonates corresponding to formula (II):
R³O-CO-OR⁴ (II)
in which
R³ and R⁴ independently of one another represent alkyl groups containing I to 10 carbon atoms,
in the presence of basic catalysts.

2. A process for the production of alkyl and/or alkenyl oligoglycoside carbonates, in which alkyl and/or alkenyl oligoglycosides corresponding to formula (I):
R¹O-(R²O)ₓ-[G]ₚ (I)
in which
R¹ is an alkyl and/or alkenyl group containing 1 to 22 carbon atoms,
R² is an alkylene group containing 2 to 4 carbon atoms,
x is 0 or a number of 1 to 30,
[G] is a sugar unit containing 5 or 6 carbon atoms and
p is a number of 1 to 10,
are condensed with dialkyl carbonates corresponding to formula (II):
R³O-CO-OR⁴ (II)
in which
R³ and R⁴ independently of one another represent alkyl groups containing 1 to 10 carbon atoms,
in the presence of basic catalysts.

3. A process as claimed in claim 2, characterized in that alkyl oligoglycosides corresponding to formula (I), in which R¹ is an alkyl group containing 8 to 18 carbon atoms, x is 0, [G] is a glucose unit and p is a number of 1.3 to 3, are used.

4. A process as claimed in claim 2, characterized in that dialkyl carbonates corresponding to formula (II), in which R³ and R⁴ are methyl and/or ethyl groups, are used.

5. A process as claimed in claim 2, characterized in that basic catalysts selected from the group consisting of alkali metal and alkaline earth metal oxides, hydroxides, carbonates and C₁₋₄ alkylates are used.

6. A process as claimed in claim 2, characterized in that the basic catalysts are used in quantities of 0.1 to 5 mole-%, based on the alkyl and/or alkenyl oligoglycosides.

7. A process as claimed in claim 2, characterized in that the alkyl and/or alkenyl oligoglycosides and the dialkyl carbonates are used in a molar ratio of 1:1 to 1:15.

8. A process as claimed in claim 2, characterized in that the condensation is carried out at the boiling temperature of the dialkyl carbonate.

9. A process as claimed in claim 2, characterized in that alcohol formed during the condensation is continuously removed from the equilibrium.

10. The use of the alkyl and/or alkenyl oligoglycoside carbonates claimed in claim 1 for the production of surface-active formulations.

## Revendications

1. Carbonates d'alkyl- et/ou alcényloligoglycosides caractérisés en ce qu'
on condense des alkyl- et/ou alcényloligoglycosides de formule (I).
R¹O-(R²O)ₓ-[G]ₚ
où :
R¹ représente un radical alkyl- et/ou alcényle de 1 à 22 atomes de carbone,
R² représente un radical alkylène de 2 à 4 atomes de carbone,
x représente O ou des nombres de 1 à 30, [G] est un radical sucre de 5 ou 6 atomes de carbone et,
p représente des nombres de 1 à 10, en présence de catalyseurs basiques avec des carbonates de dialkyle de formule (II),
R³O-CO-OR⁴
où,
R³ et R⁴ représentent indépendamment l'un de l'autre des radicaux alkyles de 1 à 10 atomes de carbone,

2. Procédé de préparation de carbonates d'alkyl- et/ou alcényloligoglycosides, où on condense des alkyl- et/ou alcényloligoglycosides de formule (I).
R¹O-(R²O)ₓ-[G]ₚ
où :
R¹ représente un radical alkyl- et/ou alcényle de 1 à 22 atomes de carbone,
R² représente un radical alkylène de 2 à 4 atomes de carbone,
x représente O ou des nombres de 1 à 30, [G] est un radical sucre de 5 ou 6 atomes de carbone et,
p représente des nombres de 1 à 10, en présence de catalyseurs basiques avec des carbonates de dialkyle de formule (Il)
R³O-CO-OR⁴
où,
R³ et R⁴ représentent indépendamment l'un de l'autre des radicaux alkyles de 1 à 10 atomes de carbone,

3. Procédé selon la revendication 2,
caractérisé en ce qu'
on utilise des alkyloligoglycosides de formule (I) où R¹ représente un radical alkyle de 8 à 18 atomes de carbone x est O, [G] est un radical glucose et p représente des nombres de 1,3 à 3.

4. Procédé selon la revendication 2,
caractérisé en ce qu'
on utilise des carbonates de dialkyle de formule (II), où R³ et R⁴ représentent des radicaux méthyle et/ou éthyle.

5. Procédé selon la revendication 2,
caractérisé en ce qu'
on utilise des catalyseurs basiques qu'on choisit dans le groupe des oxydes, des hydroxydes, des carbonates et des alkylates C₁-C₄ de métaux alcalins et alcalino-terreux;

6. Procédé selon la revendication 2,
caractérisé en ce qu'
on utilise des catalyseurs basiques en des quantités de 0,1 à 5 % molaire, par rapport aux alkyl- et/ou alcényloligoglycosides.

7. Procédé selon la revendication 2,
caractérisé en ce qu'
on utilise les alkyl- et/ou alcényloligoglycosides et les carbonates de dialkyle en un rapport molaire de 1:1 à 1:15.

8. Procédé selon la revendication 2,
caractérisé en ce qu'
on réalise la condensation à la température d'ébullition du carbonate de dialkyle.

9. Procédé selon la revendication 2,
caractérisé en ce qu'
on élimine en continu de l'équilibre l'alcool formé au cours de la condensation.

10. Utilisation de carbonates d'alkyl- et/ou d'alcényloligoglycosides selon la revendications 1 pour préparer des agents tensioactifs.
